# EUROPÄISCHE PATENTANMELDUNG

(11) **EP 4 474 034 A1**
(43) Veröffentlichungstag der Anmeldung: **11.12.2024**
(21) Anmeldenummer: 23178025.5
(22) Anmeldetag: 07.06.2023
(51) Int. Cl.: B01D 5/00, C07C 29/151, C07C 31/04

(54) **ABSCHEIDUNG VON SYNTHETISIERTEM METHANOL**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt am Main (DE); Strozyk, Michael, 60439 Frankfurt am Main (DE); Peña, Vincent, 60439 Frankfurt am Main (DE)
(74) Vertreter: Air Liquide

(57) **Zusammenfassung**

Verfahren zur Synthese von Methanol mit einer Synthesereaktoranordnung (1), wobei mit der Synthesereaktoranordnung (1) erzeugtes Methanol mit einer Abscheideeinheit (2) aus einem Auslassstrom der Synthesereaktoranordnung (1) abgeschieden wird, wobei die Abscheideeinheit (2) mehrere strömungstechnisch parallelgeschaltete Abscheideeinrichtungen (3.1 bis 3.5) aufweist, wobei der Auslassstrom derart auf die Abscheideeinrichtungen (3.1 bis 3.5) aufgeteilt wird, dass durch eine der Abscheideeinrichtungen (3.1 bis 3.5) der gesamte Auslassstrom geleitet wird oder durch mehrere der Abscheideeinrichtungen (3.1 bis 3.5) jeweils ein Teil des Auslassstroms geleitet wird, und wobei eine Aufteilung des Auslassstroms auf die Abscheideeinrichtungen (3.1 bis 3.5) in Abhängigkeit von einer Volumenströmungsrate des Auslassstroms eingestellt wird.

## Beschreibung

Die Erfindung betrifft ein Verfahren und eine Anordnung zur Synthese von Methanol. In dem Kontext ist die Erfindung insbesondere auf die Abscheidung des Methanols aus einem Auslassstrom der verwendeten Synthesereaktoranordnung gerichtet.

Eines der Edukte der Methanol-Synthese ist Wasserstoff. Für eine möglichst umweltfreundliche Methanol-Synthese ist es wünschenswert, den Wasserstoff aus einer Wasserelektrolyse zu verwenden, welche mit erneuerbaren Energien betrieben wird. Erneuerbare Energien schwanken allerdings, so dass auch die Verfügbarkeit von Wasserstoff aus einer entsprechenden Quelle schwankt. Mit der Verfügbarkeit des Edukts Wasserstoff schwankt auch ein Auslassstrom der Synthesereaktoranordnung.

Das erzeugte Methanol ist in dem Auslassstrom enthalten und wird üblicherweise mit einem Abscheider aus dem Auslassstrom abgeschieden. Bekannte Abscheider funktionieren allerdings nur in einem vergleichsweise begrenzten Bereich von Strömungsraten des Auslassstroms. Der effektive Einsatz eines Abscheiders setzt eine Strömungsgeschwindigkeit des in den Abscheider eintretenden Auslassstroms innerhalb eines definierten Wertebereichs voraus, damit die Kondensattröpfchen im Abscheider abgefangen werden können. Ist der Auslassstrom außerhalb eines geeigneten Bereichs sinkt die Trennleistung des Abscheiders und das Risiko steigt, dass Flüssigkeitströpfchen den Abscheider passieren. Das kann einen nachgeschalteten Kompressor beschädigen.

Die Abscheidung des Methanols ist also schwierig, wenn der Auslassstrom der Synthesereaktoranordnung schwankt, wie dies insbesondere bei der Methanolerzeugung auf Basis von Wasserstoff der Fall ist, welcher mit erneuerbaren Energien erzeugt wurde.

Aufgabe der vorliegenden Erfindung ist es, eine Möglichkeit zu schaffen, Methanol auch bei schwankendem Reaktoraustrittsstrom aufgrund einer schwankenden Eduktzufuhr zuverlässig aus dem Auslassstrom abzuscheiden.

Diese Aufgabe wird gelöst mit dem Verfahren und der Vorrichtung gemäß den unabhängigen Ansprüchen. Weitere vorteilhafte Ausgestaltungen sind in den abhängigen Ansprüchen angegeben. Die in den Ansprüchen und in der Beschreibung dargestellten Merkmale sind in beliebiger, technologisch sinnvoller Weise miteinander kombinierbar.

Erfindungsgemäß wird ein Verfahren zur Synthese von Methanol mit einer Synthesereaktoranordnung vorgestellt, wobei mit der Synthesereaktoranordnung erzeugtes Methanol mit einer Abscheideeinheit aus einem Auslassstrom der Synthesereaktoranordnung abgeschieden wird, wobei die Abscheideeinheit mehrere strömungstechnisch parallelgeschaltete Abscheideeinrichtungen aufweist, wobei der Auslassstrom derart auf die Abscheideeinrichtungen aufgeteilt wird, dass durch eine der Abscheideeinrichtungen der gesamte Auslassstrom geleitet wird oder durch mehrere der Abscheideeinrichtungen jeweils ein Teil des Auslassstroms geleitet wird, und wobei eine Aufteilung des Auslassstroms auf die Abscheideeinrichtungen in Abhängigkeit von einer Volumenströmungsrate des Auslassstroms eingestellt wird.

Mit dem beschriebenen Verfahren kann Methanol aus Edukten wie Wasserstoff einerseits und Kohlendioxid und/oder Kohlenmonoxid andererseits hergestellt werden. Das kann mit der Synthesereaktoranordnung auf für sich genommen bekannte Weise erfolgen. Die Edukte können der Synthesereaktoranordnung über einen Feedgasstrom aus einer Rohgasquelle zugeführt werden. Für das beschriebene Verfahren kommt es nicht darauf an, ob die Edukte der Synthesereaktoranordnung einzeln oder als Gemisch zugeführt werden. Vorzugsweise ist eines der Edukte Wasserstoff, welcher aus einer mit erneuerbaren Energien betriebenen Elektrolyse gewonnen wird.

Die Produkte der in der Synthesereaktoranordnung ablaufenden Reaktion werden zusammen mit nicht reagierten Edukten als ein Auslassstrom aus der Synthesereaktoranordnung ausgelassen. Der Auslassstrom enthält also das erzeugte Methanol. Um dieses Methanol für eine weitere Verarbeitung zugänglich zu machen, wird das Methanol aus dem Auslassstrom abgeschieden. Dies erfolgt mit einer Abscheideeinheit. Die Abscheideeinheit kann insbesondere dazu eingerichtet sein, eine Flüssigkeit und ein Gas voneinander zu trennen. Die Abscheideeinheit dient in dem Fall der Abtrennung eines Kondensats aus einem zweiphasigen Gas-Flüssigkeits-Strom.

Insoweit hat die Abscheideeinheit die gleiche Funktion wie ein bei einer herkömmlichen Anordnung zur Synthese von Methanol üblicherweise verwendeter Abscheider. Die Abscheideeinheit weist vorzugsweise einen Vorabscheider und einen Hauptabscheider auf. Der Vorabscheider und der Hauptabscheider werden vorzugsweise nacheinander vertikal von unten nach oben durchströmt, sodass abgeschiedenes Kondensat mit der Schwerkraft nach unten fließt, wo das Rohmethanol am Boden des Abscheidebehälters gesammelt wird, um dann ausgeleitet zu werden.

Die bei dem beschriebenen Verfahren verwendete Abscheideeinheit unterscheidet sich im Aufbau von einem herkömmlichen Abscheider. Insbesondere weist die Abscheideeinheit mehrere strömungstechnisch parallelgeschaltete Abscheideeinrichtungen auf. Strömungstechnisch parallelgeschaltet bedeutet, dass ein bestimmter Teil des Auslassstroms durch genau eine der Abscheideeinrichtungen strömt. Stromauf der Abscheideeinrichtungen wird der Auslassstrom in einen oder mehrere Teile aufgeteilt. Jeder der Teile wird durch genau eine der Abscheideeinrichtungen geleitet. Stromab der Abscheideeinrichtungen können die einzelnen Teile des Auslassstroms wieder zusammengeführt werden. Es versteht sich, dass dies nur für die nicht mit den Abscheideeinrichtungen aus dem Auslassstrom abgeschiedenen flüssigen Anteile des Auslassstroms gilt.

Die Abscheideeinheit umfasst die Abscheideeinrichtungen. Die Abscheideeinheit bildet also insoweit eine Einheit, als dass die Abscheideeinrichtungen unter den Oberbegriff der Abscheideeinheit zusammengefasst werden. Die Abscheideeinheit ist also als die Summe der Abscheideeinrichtungen definiert. Es ist nicht erforderlich, dass die Abscheideeinheit ein gesondertes Bauteil ist, in welche alle Abscheideeinrichtungen integriert sind. Es genügt, dass die Abscheideeinrichtungen derart zusammenwirken, dass der Auslassstrom wie beschrieben auf diese aufgeteilt werden kann und dass die Abscheideeinrichtungen dadurch gemeinsam die hierin beschriebene Funktion der Abscheideeinheit erfüllen können.

Die Abscheideeinheit hat vorzugsweise genau zwei oder genau vier Abscheideeinrichtungen.

Der Auslassstrom wird derart auf die Abscheideeinrichtungen aufgeteilt, dass durch eine der Abscheideeinrichtungen der gesamte Auslassstrom geleitet wird oder durch mehrere der Abscheideeinrichtungen jeweils ein Teil des Auslassstroms geleitet wird. Aufteilen bedeutet also, dass jeder der Abscheideeinrichtungen ein Anteil des Auslassstroms zugewiesen wird. Für jede der Abscheideeinrichtungen kann dieser Anteil jeweils zwischen 0 und 100 % liegen, wobei die Summe der Anteile aller Abscheideeinrichtungen 100 % ergibt. Das Aufteilen des Auslassstroms auf die Abscheideeinrichtungen umfasst also auch den Grenzfall, in dem einer oder mehreren der Abscheideeinrichtungen jeweils ein Anteil von 0 % zugewiesen wird, so dass diese Abscheideeinrichtung(en) gar nicht von dem Auslassstrom durchströmt werden. Auch umfasst das Aufteilen des Auslassstroms auf die Abscheideeinrichtungen den Grenzfall, in dem einer der Abscheideeinrichtungen ein Anteil von 100 % zugewiesen wird. Für alle anderen Abscheideeinrichtungen muss der Anteil in dem Fall 0 % sein. Dass das Aufteilen auch die Grenzfälle umfasst, wird dadurch klargestellt, dass nicht bloß von der Aufteilung des Auslassstroms die Rede ist, sondern davon, dass der Auslassstrom derart auf die Abscheideeinrichtungen aufgeteilt wird, dass durch eine der Abscheideeinrichtungen der gesamte Auslassstrom geleitet wird oder durch mehrere der Abscheideeinrichtungen jeweils ein Teil des Auslassstroms geleitet wird.

Die Aufteilung des Auslassstroms auf die Abscheideeinrichtungen wird in Abhängigkeit von einer Volumenströmungsrate des Auslassstroms eingestellt. Damit kann das beschriebene Verfahren besonders gut auf Schwankungen im Auslassstrom reagieren. Insbesondere kann erreicht werden, dass Schwankungen der jeweiligen Strömungsgeschwindigkeit in den einzelnen Abscheideeinrichtungen vergleichsweise gering sind. Das folgende Beispiel verdeutlicht dies. Dabei hat die Abscheideeinheit drei identische Abscheideeinrichtungen. Im Falle einer geringen Volumenströmungsrate des Auslassstroms wird der gesamte Auslassstrom nur durch eine der drei Abscheideeinrichtungen geleitet- welche dies ist, ist unerheblich. Angenommen, die Volumenströmungsrate des Auslassstroms verdoppelt sich. Wird dann eine zweite der Abscheideeinrichtungen hinzugezogen, ist die Strömungsgeschwindigkeit in beiden aktiven Abscheideeinrichtungen so groß wie zuvor in der einzigen aktiven Abscheideeinrichtung. Angenommen, die Volumenströmungsrate des Auslassstroms erhöht sich auf das Dreifache des Ausgangswertes. Wird dann auch die dritte der Abscheideeinrichtungen hinzugezogen, ist die Strömungsgeschwindigkeit in allen drei Abscheideeinrichtungen so groß wie zuvor in der einzigen beziehungsweise in den beiden aktiven Abscheideeinrichtungen.

Die Aufteilung des Auslassstroms auf die Abscheideeinrichtungen umfasst einerseits die Frage, auf wie viele der Abscheideeinrichtungen der Auslassstrom aufgeteilt wird. Diese Abscheideeinrichtungen können als aktive Abscheideeinrichtungen bezeichnet werden. Andererseits ist aber auch die Frage umfasst, wie der Auslassstrom auf die aktiven Abscheideeinrichtungen aufgeteilt wird. Bevorzugt ist beispielsweise, dass die Anzahl der aktiven Abscheideeinrichtungen in Abhängigkeit von der Volumenströmungsrate des Auslassstroms gewählt wird, und dass der Auslassstrom zu gleichen Teilen auf die aktiven Abscheideeinrichtungen aufgeteilt wird. Alternativ ist es bevorzugt, dass die Anzahl der aktiven Abscheideeinrichtungen in Abhängigkeit von der Volumenströmungsrate des Auslassstroms gewählt wird, und dass maximal eine der aktiven Abscheideeinrichtungen nur mit einem Teil der Maximalkapazität dieser Abscheideeinrichtung betrieben wird. Es wird also immer dann eine weitere Abscheideeinrichtung hinzugezogen, wenn die bereits aktiven Abscheideeinrichtungen voll ausgelastet sind. Möglich ist aber auch, dass die Anzahl der aktiven Abscheideeinrichtungen in Abhängigkeit von der Volumenströmungsrate des Auslassstroms gewählt wird, und dass zugelassen wird, dass sich der Auslassstrom frei auf die aktiven Abscheideeinrichtungen verteilt. Es wird also die Aufteilung auf die aktiven Abscheideeinrichtungen hingenommen, die sich von selbst einstellt.

Die Aufteilung des Auslassstroms auf die Abscheideeinrichtungen wird vorzugsweise derart in Abhängigkeit von der Volumenströmungsrate des Auslassstroms eingestellt, dass eine Strömungsgeschwindigkeit des Abgases in jeder der Abscheideeinrichtungen innerhalb eines vorgegebenen Bereichs liegt. Durch die Aufteilung des Auslassstroms auf eine oder mehrere der Abscheideeinrichtungen kann der vorgegebene Bereich für die einzelnen Abscheideeinrichtungen vergleichsweise klein sein, insbesondere im Vergleich zu der Spannweite von Strömungsgeschwindigkeiten, welche ein herkömmlicher Abscheider mit nur einer einzelnen Abscheideeinrichtung in einer vergleichbaren Situation ausgesetzt wäre.

Die Abscheideeinrichtungen haben jeweils eine Abscheidekapazität. Diese kann sich beispielsweise aus einem Strömungsquerschnitt ergeben. Die Aufteilung des Auslassstroms auf die Abscheideeinrichtungen wird vorzugsweise derart in Abhängigkeit von der Volumenströmungsrate des Auslassstroms eingestellt, dass eine Summe der Abscheidekapazitäten der aktiven Abscheideeinrichtungen umso größer ist, je größer die Volumenströmungsrate des Auslassstroms ist, und umgekehrt. Insbesondere ist es bevorzugt, dass die Aufteilung des Auslassstroms auf die Abscheideeinrichtungen derart in Abhängigkeit von der Volumenströmungsrate des Auslassstroms eingestellt wird, dass eine Summe der Strömungsquerschnitte der aktiven Abscheideeinrichtungen umso größer ist, je größer die Volumenströmungsrate des Auslassstroms ist, und umgekehrt.

Die Abscheideeinrichtungen haben jeweils einen Arbeitsbereich. Der Arbeitsbereich kann insbesondere als ein Bereich der Volumenströmungsraten definiert sein, mit welcher die jeweilige Abscheideeinrichtung beaufschlagt werden kann. Die Aufteilung des Auslassstroms auf die Abscheideeinrichtungen wird vorzugsweise derart in Abhängigkeit der Volumenströmungsrate des Auslassstroms eingestellt, dass die Volumenströmungsrate des Auslassstroms in einen sich aus den Arbeitsbereichen der aktiven Abscheideeinrichtungen zusammengesetzten Arbeitsbereich der Abscheideeinheit fällt.

Dass der Auslassstrom derart auf die Abscheideeinrichtungen aufgeteilt wird, dass durch eine der Abscheideeinrichtungen der gesamte Auslassstrom geleitet wird oder durch mehrere der Abscheideeinrichtungen jeweils ein Teil des Auslassstroms geleitet wird, wobei eine Aufteilung des Auslassstroms auf die Abscheideeinrichtungen in Abhängigkeit von einer Volumenströmungsrate des Auslassstroms eingestellt wird, kann auch dadurch ausgedrückt werden, dass durch eine der Abscheideeinrichtungen der gesamte Auslassstrom geleitet wird oder durch mehrere der Abscheideeinrichtungen jeweils ein Teil des Auslassstroms geleitet wird, wobei in Abhängigkeit von einer Volumenströmungsrate des Auslassstroms eingestellt wird, wie groß die durch die einzelnen Abscheideeinrichtungen geleiteten Anteile des Auslassstroms jeweils sind. Die Anteile können jeweils zwischen 0 und 100 % liegen.

Die Abscheideeinheit weist vorzugsweise einen Vorabscheider und einen Hauptabscheider auf. Insoweit gibt es verschiedene Möglichkeiten. Der Hauptabscheider kann die Abscheideeinrichtungen aufweisen. Dem so zusammengesetzten Hauptabscheider kann ein gemeinsamer Vorabscheider vorgeschaltet sein. Alternativ kann auch der Vorabscheider unterteilt sein. Einem aus mehreren Abscheideeinrichtungen zusammengesetzten Hauptabscheider können mehrere Vorabscheideeinrichtungen vorgeschaltet sein. Alternativ können die Abscheideeinrichtungen jeweils ein Hauptabscheideelement und ein Vorabscheideelement aufweisen. Die Hauptabscheideelemente der Abscheideeinrichtungen entsprechen in dem Fall in ihrer Gesamtheit funktional einem Hauptabscheider und die Vorabscheideelemente der Abscheideeinrichtungen entsprechen in dem Fall in ihrer Gesamtheit funktional einem Vorabscheider. Allgemein kommt es für die Funktionsweise des beschriebenen Verfahrens nicht darauf an, ob der Abscheider in Bezug auf die Hauptabscheidefunktion und/oder in Bezug auf die Vorabscheidefunktion in die Abscheideeinrichtungen unterteilt ist. Bevorzugt ist jedenfalls der Hauptabscheider auf mehrere Abscheideeinrichtungen aufgeteilt. Die Abscheideeinrichtungen können insbesondere als Kompartimente des Hauptabscheiders ausgebildet sein.

In einer bevorzugten Ausführungsform des Verfahrens wird die Aufteilung des Auslassstroms auf die Abscheideeinrichtungen in Abhängigkeit der Volumenströmungsrate des Auslassstroms eingestellt, indem eine Anzahl der Abscheideeinrichtungen eingestellt wird, durch welche jeweils zumindest ein Teil des Auslassstroms geleitet wird.

Der Auslassstrom wird derart auf die Abscheideeinrichtungen aufgeteilt, dass durch eine der Abscheideeinrichtungen der gesamte Auslassstrom geleitet wird oder durch mehrere der Abscheideeinrichtungen jeweils ein Teil des Auslassstroms geleitet wird. Je größer die Volumenströmungsrate des Auslassstroms ist, umso größer ist vorzugsweise die Anzahl der aktiven Abscheideeinrichtungen. Bei einer kleinen Volumenströmungsrate ist es daher bevorzugt, dass der gesamte Auslassstrom durch eine einzige der Abscheideeinrichtungen geleitet wird. Mit steigender Volumenströmungsrate können dann nach und nach weitere der Abscheideeinrichtungen hinzugenommen werden.

In einer weiteren bevorzugten Ausführungsform des Verfahrens wird die Aufteilung des Auslassstroms auf die Abscheideeinrichtungen in Abhängigkeit von der Volumenströmungsrate des Auslassstroms eingestellt, indem für die Abscheideeinrichtungen jeweils ein Anteil des Auslassstroms eingestellt wird, welcher durch die jeweilige Abscheideeinrichtung geleitet wird.

Der Anteil kann für jede der Abscheideeinrichtungen jeweils zwischen 0 und 100 % liegen. Die Einstellung des Anteils umfasst also auch die Entscheidung, ob eine Abscheideeinrichtung aktiv oder inaktiv ist.

In einer weiteren bevorzugten Ausführungsform des Verfahrens hat der Auslassstrom in einem ersten Fall eine kleinere Volumenströmungsrate als in einem zweiten Fall, wobei der Auslassstrom im ersten Fall durch weniger der Abscheideeinrichtungen zumindest teilweise geleitet wird als im zweiten Fall.

Allgemein ist es bevorzugt, dass umso mehr der Abscheideeinrichtungen aktiv sind, je größer die Volumenströmungsrate des Auslassstroms ist. Diese Abhängigkeit ist naturgemäß nicht linear, sondern stufenartig.

In einer weiteren bevorzugten Ausführungsform des Verfahrens hat der Auslassstrom in einem ersten Fall eine kleinere Volumenströmungsrate als in einem zweiten Fall, wobei eine erste der Abscheideeinrichtungen einen kleineren Strömungsquerschnitt hat als eine zweite der Abscheideeinrichtungen, und wobei der Auslassstrom im ersten Fall vollständig durch die erste Abscheideeinrichtung und im zweiten Fall vollständig durch die zweite Abscheideeinrichtung geleitet wird.

In der vorliegenden Ausführungsform sind die Abscheideeinrichtungen unterschiedlich groß. Das drückt sich dadurch aus, dass die erste Abscheideeinrichtung einen kleineren Strömungsquerschnitt hat als die zweite Abscheideeinrichtung. Bei kleiner Volumenströmungsrate des Auslassstroms wird daher die erste Abscheideeinrichtung verwendet. Bei größerem Volumenströmungsrate des Auslassstroms wird die größere zweite Abscheideeinrichtung verwendet. Von der ersten Abscheideeinrichtung zur zweiten Abscheideeinrichtung kann beispielsweise gewechselt werden, wenn die Volumenströmungsrate des Auslassstroms einen Grenzwert überschreitet.

In einer weiteren bevorzugten Ausführungsform des Verfahrens hat der Auslassstrom in einem dritten Fall eine größere Volumenströmungsrate als in dem zweiten Fall, wobei der Auslassstrom im dritten Fall jeweils zumindest teilweise durch die erste Abscheideeinrichtung und durch die zweite Abscheideeinrichtung geleitet wird. Von der zweiten Abscheideeinrichtung zur Kombination aus der ersten Abscheideeinrichtung und der zweiten Abscheideeinrichtung kann beispielsweise gewechselt werden, wenn die Volumenströmungsrate des Auslassstroms einen weiteren Grenzwert überschreitet.

Diese Ausführungsform baut auf der zuvor beschriebenen Ausführungsform auf. Ist die Volumenströmungsrate des Auslassstroms auch für die größere zweite Abscheideeinrichtung zu groß, kann die kleinere erste Abscheideeinrichtung hinzugeschaltet werden.

In einer weiteren bevorzugten Ausführungsform des Verfahrens wird mit der Synthesereaktoranordnung Methanol mit einer Erzeugungsrate von bis zu 5.000 Tonnen Reinmethanol pro Tag erzeugt, besonders bevorzugt von bis zu 1.000 Tonnen pro Tag. Das bedeutet, dass 5.000 t Methanolmoleküle erzeugt werden.

Als ein weiterer Aspekt der Erfindung wird eine Anordnung zur Synthese von Methanol vorgestellt, welche eine Synthesereaktoranordnung und eine an die Synthesereaktoranordnung angebundene Abscheideeinheit umfasst, wobei die Abscheideeinheit dazu eingerichtet ist, mit der Synthesereaktoranordnung erzeugtes Methanol aus einem Auslassstrom der Synthesereaktoranordnung abzuscheiden, und wobei die Abscheideeinheit mehrere strömungstechnisch parallelgeschaltete Abscheideeinrichtungen aufweist.

Die beschriebenen Vorteile und Merkmale des Verfahrens sind auf die Anordnung anwendbar und übertragbar, und umgekehrt. Die Anordnung ist vorzugsweise zum Betrieb gemäß dem Verfahren eingerichtet. Das Verfahren wird vorzugsweise mit der Anordnung durchgeführt.

In einer bevorzugten Ausführungsform weist die Anordnung weiterhin eine Steuereinheit auf, welche dazu eingerichtet ist, den Auslassstrom derart auf die Abscheideeinrichtungen aufzuteilen, dass durch eine der Abscheideeinrichtungen der gesamte Auslassstrom geleitet wird oder durch mehrere der Abscheideeinrichtungen jeweils ein Teil des Auslassstroms geleitet wird, und eine Aufteilung des Auslassstroms auf die Abscheideeinrichtungen in Abhängigkeit von einer Volumenströmungsrate des Auslassstroms einzustellen.

In dieser Ausführungsform kann das zuvor beschriebene Verfahren automatisiert durchgeführt werden. Die Steuereinheit ist vorzugsweise zur Durchführung des beschriebenen Verfahrens eingerichtet.

Die Verwendung einer wie beschrieben ausgestalteten Steuereinheit ist allerdings nicht zwingend. In einer Alternative kann die Vorrichtung auch ohne Steuereinheit entsprechend des beschriebenen Verfahrens eingesetzt werden, beispielsweise indem die Aufteilung des Auslassstroms auf die Abscheideeinrichtungen manuell durchgeführt wird. Zur Erreichung der hierin beschriebenen Vorteile ist es nicht erforderlich, dass die Aufteilung automatisiert erfolgt. Es genügt, dass die Vorrichtung die Aufteilung des Auslassstroms ermöglicht. Das ist der Fall, wenn die Abscheideeinheit wie beschrieben im Unterschied zu einem herkömmlichen Abscheider mehrere strömungstechnisch parallelgeschaltete Abscheideeinrichtungen aufweist.

In einer weiteren bevorzugten Ausführungsform der Anordnung ist ein jeweiliger Strömungsquerschnitt der Abscheideeinrichtungen gleich groß.

In dieser Ausführungsform kann die Aufteilung des Auslassstroms auf die Abscheideeinrichtungen in Abhängigkeit der Volumenströmungsrate des Auslassstroms insbesondere dadurch eingestellt werden, dass die Anzahl der aktiven Abscheideeinrichtungen in Abhängigkeit von der Volumenströmungsrate des Auslassstroms gewählt wird. Diese Ausführungsform ist besonders einfach in der Umsetzung, da mit der gleichen Größe der Abscheideeinrichtungen ein Freiheitsgrad bei der Aufteilung des Auslassstroms wegfällt.

In einer weiteren bevorzugten Ausführungsform der Anordnung hat eine erste der Abscheideeinrichtungen einen kleineren Strömungsquerschnitt als eine zweite der Abscheideeinrichtungen.

In dieser Ausführungsform kann die Aufteilung des Auslassstroms auf die Abscheideeinrichtungen in Abhängigkeit der Volumenströmungsrate des Auslassstroms insbesondere dadurch eingestellt werden, dass die Anzahl der aktiven Abscheideeinrichtungen in Abhängigkeit von der Volumenströmungsrate des Auslassstroms gewählt wird und/oder dass in Abhängigkeit von der Volumenströmungsrate gewählt wird, welche der Abscheideeinrichtungen aktiv sind. Beispielsweise können bei einer größeren Volumenströmungsrate größere Abscheideeinrichtungen bevorzugt werden. Diese Ausführungsform ist gegenüber der zuvor beschriebenen Ausführungsform flexibler.

In einer weiteren bevorzugten Ausführungsform der Anordnung sind die Abscheideeinrichtungen für das gleiche Abscheideprinzip eingerichtet.

In dieser Ausführungsform kann die Aufteilung des Auslassstroms auf die Abscheideeinrichtungen in Abhängigkeit von der Volumenströmungsrate des Auslassstroms insbesondere dadurch eingestellt werden, dass die Anzahl der aktiven Abscheideeinrichtungen in Abhängigkeit von der Volumenströmungsrate des Auslassstroms gewählt wird. Diese Ausführungsform ist besonders einfach in der Umsetzung, da mit dem gleichen Abscheideprinzip ein Freiheitsgrad bei der Aufteilung des Auslassstroms wegfällt.

In einer weiteren bevorzugten Ausführungsform der Anordnung ist eine erste der Abscheideeinrichtungen für ein anderes Abscheideprinzip eingerichtet als eine zweite der Abscheideeinrichtungen.

In dieser Ausführungsform kann die Aufteilung des Auslassstroms auf die Abscheideeinrichtungen in Abhängigkeit der Volumenströmungsrate des Auslassstroms insbesondere dadurch eingestellt werden, dass die Anzahl der aktiven Abscheideeinrichtungen in Abhängigkeit von der Volumenströmungsrate des Auslassstroms gewählt wird und/oder dass in Abhängigkeit von der Volumenströmungsrate gewählt wird, welche der Abscheideeinrichtungen aktiv sind. Beispielsweise können bei einer größeren Volumenströmungsrate die Abscheideeinrichtungen bevorzugt werden, deren Abscheideprinzip für eine größere Volumenströmungsrate geeignet sind. Diese Ausführungsform ist gegenüber der zuvor beschriebenen Ausführungsform flexibler.

In einer weiteren bevorzugten Ausführungsform der Anordnung sind zumindest einige der Abscheideeinrichtungen in einem gemeinsamen Gehäuse angeordnet.

Besonders bevorzugt sind alle der Abscheideeinrichtungen in dem gemeinsamen Gehäuse angeordnet.

Die Zusammenfassung der Abscheideeinrichtungen in einem gemeinsamen Gehäuse ist aus praktischen Gründen vorteilhaft. Die Abscheideeinheit ist in dem Fall als ein gesondertes Bauteil ausgebildet. Dieses kann in eine im Übrigen herkömmlich ausgebildete Anordnung zur Synthese von Methanol anstelle eines herkömmlichen Abscheiders eingebunden werden. Die Abscheideeinheit kann insoweit auch als ein Abscheider aufgefasst werden, welcher einen gegenüber einem herkömmlichen Abscheider erweiterten Arbeitsbereich hat. Das gilt umso mehr, wenn die hierin beschriebene Steuereinheit in die Abscheideeinheit integriert ist oder an diese angebunden ist.

In einer weiteren bevorzugten Ausführungsform der Anordnung zumindest einige der Abscheideeinrichtungen in unterschiedlichen Gehäusen angeordnet sind.

Besonders bevorzugt sind alle der Abscheideeinrichtungen in unterschiedlichen Gehäusen angeordnet. Möglich ist aber auch verschiedene Kombinationen mit der zuvor beschriebenen Ausführungsform.

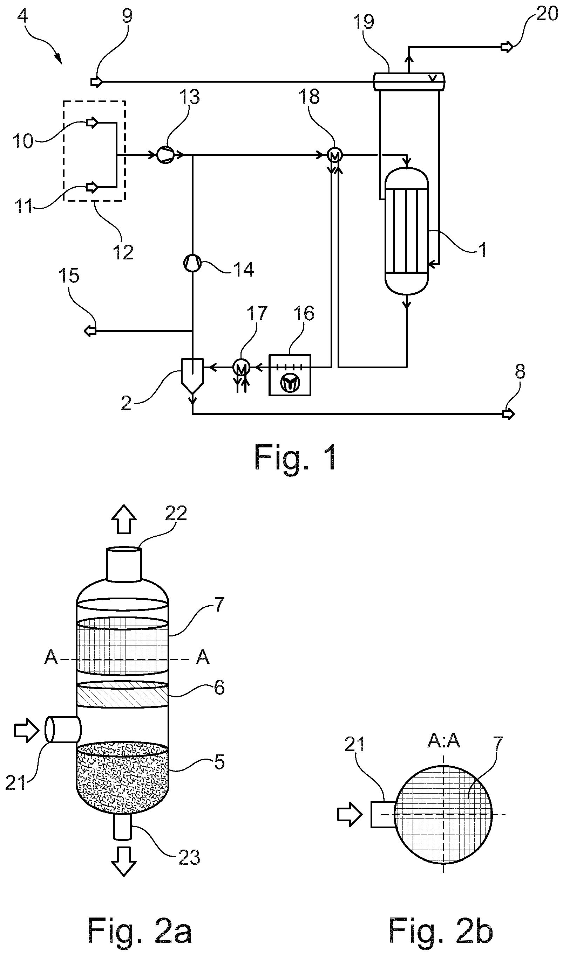

Die Erfindung wird nachfolgend anhand der Figuren 1 und 4 bis 8b näher erläutert. Die Figuren 1 und 4 bis 8b zeigen besonders bevorzugte Ausführungsbeispiele, auf welche die Erfindung jedoch nicht begrenzt ist. Die Figuren und die darin dargestellten Größenverhältnisse sind nur schematisch. Es zeigen:
- Fig. 1:: eine erfindungsgemäße Anordnung zur Synthese von Methanol,
- Fig. 2a:: eine perspektivische Ansicht eines aus dem Stand der Technik bekannten Abscheiders,
- Fig. 2b:: eine Schnittdarstellung von oben auf den aus dem Stand der Technik bekannten Abscheider aus Fig. 2a,
- Fig. 3:: eine schematische Darstellung des aus dem Stand der Technik bekannten Abscheiders aus Fig. 2a und 2b,
- Fig. 4:: eine schematische Darstellung einer ersten Ausführungsform einer Abscheideeinheit der erfindungsgemäßen Anordnung aus Fig. 1,
- Fig. 5a bis 5c:: Veranschaulichungen einer zweiten Ausführungsform der Abscheideeinheit der erfindungsgemäßen Anordnung aus Fig. 1,
- Fig. 6a und 6b:: Veranschaulichungen einer dritten Ausführungsform der Abscheideeinheit der erfindungsgemäßen Anordnung aus Fig. 1,
- Fig. 7a und 7b:: Veranschaulichungen einer vierten Ausführungsform der Abscheideeinheit der erfindungsgemäßen Anordnung aus Fig. 1,
- Fig. 8a und 8b:: Veranschaulichungen einer fünften Ausführungsform der Abscheideeinheit der erfindungsgemäßen Anordnung aus Fig. 1.

Fig. 1 zeigt eine Anordnung 4 zur Synthese von Methanol. Die Anordnung 4 umfasst eine Rohgasquelle 12, eine Synthesereaktoranordnung 1 und eine daran angebundene Abscheideeinheit 2. Die Abscheideeinheit 2 ist dazu eingerichtet, mit der Synthesereaktoranordnung 1 erzeugtes Methanol aus einem Auslassstrom der Synthesereaktoranordnung 1 abzuscheiden. Das abgeschiedene Methanol kann an einem Methanolauslass 8 ausgelassen werden. Die Rohgasquelle 12 umfasst eine H₂-Quelle 11 und eine CO₂-Quelle 10. Die Rohgasquelle 12, die Synthesereaktoranordnung 1 und die Abscheideeinheit 2 sind über ein Rohrleitungssystem miteinander verbunden. Weiterhin umfasst die Anordnung 4 eine Wasserzuleitung 9, welche mit einer Dampftrommel 19 verbunden ist. Von der Dampftrommel 19 ausgehender Dampf kann über einen Dampfauslass 20 ausgelassen werden.

In das Rohrleitungssystem sind weiterhin ein erster Kühler 16, ein zweiter Kühler 17, ein Wärmetauscher 18, ein erster Verdichter 13 und ein zweiter Verdichter 14 eingebunden. Im Falle eines Überdrucks im Rohrleitungssystem kann Gas über einen Überdruckablass 15 abgelassen werden.

Fig. 2a und 2b zeigen einen aus dem Stand der Technik bekannten Abscheider. Dieser könnte in einer Anordnung analog zu der aus Fig. 1 anstelle der Abscheideeinheit 2 eingesetzt werden. Der Abscheider weist einen Einlass 21, einen Gasauslass 22 und einen Flüssigkeitsauslass 23 auf. In einem Gehäuse 5 des Abscheiders sind ein Vorabscheider 6 und ein Hauptabscheider 7 angeordnet. Tritt ein Auslassstrom einer Synthesereaktoranordnung über den Einlass 21 in den Abscheider ein, werden durch den Vorabscheider 6 und den Hauptabscheider 7 Flüssigkeit und Gas in dem Auslassstrom getrennt. Die Flüssigkeit sammelt sich im unteren Bereich des Gehäuses 5 und kann über den Flüssigkeitsauslass 23 ausgelassen werden. Das Gas steigt im Gehäuse 5 auf und kann über den Gasauslass 22 ausgelassen werden. Bei dem bekannten Abscheider gibt es nur die eine Möglichkeit, den gesamten Auslassstrom durch den Abscheider zu leiten. Dies ist bei schwankendem Auslassstrom nachteilig.

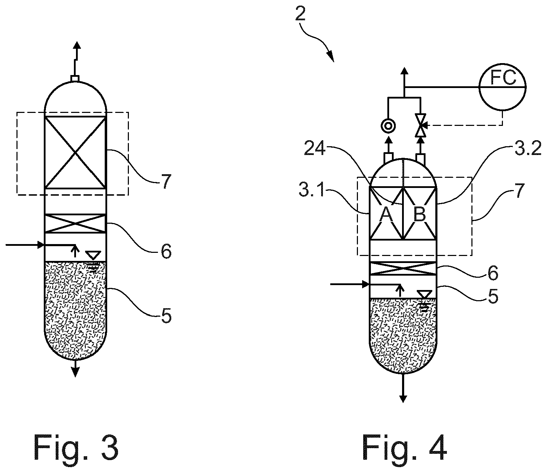

Fig. 3 zeigt eine schematische Darstellung des aus dem Stand der Technik bekannten Abscheiders aus Fig. 2a und 2b.

Fig. 4 zeigt analog zu Fig. 3 eine schematische Darstellung einer ersten Ausführungsform einer Abscheideeinheit 2 der erfindungsgemäßen Anordnung 4 aus Fig. 1. Auch die Abscheideeinheit 2 hat ein Gehäuse 5, in welchem ein Vorabscheider 6 und ein Hauptabscheider 7 ausgebildet sind. Allerdings ist der Hauptabscheider 7 in diesem Fall durch eine erste Abscheideeinrichtung 3.1 und eine zweite Abscheideeinrichtung 3.2 gebildet, welche durch eine Trennwand 24 voneinander getrennt sind.

Die Abscheideeinrichtungen 3.1,3.2 sind strömungstechnisch parallelgeschaltet. Dadurch kann der Auslassstrom der Synthesereaktoranordnung 1 derart auf die Abscheideeinrichtungen 3.1,3.2 aufgeteilt werden, dass durch eine der Abscheideeinrichtungen 3.1,3.2 der gesamte Auslassstrom geleitet wird oder durch beide der Abscheideeinrichtungen 3.1,3.2 jeweils ein Teil des Auslassstroms geleitet wird, wobei eine Aufteilung des Auslassstroms auf die Abscheideeinrichtungen 3.1,3.2 in Abhängigkeit von einer Volumenströmungsrate des Auslassstroms eingestellt wird.

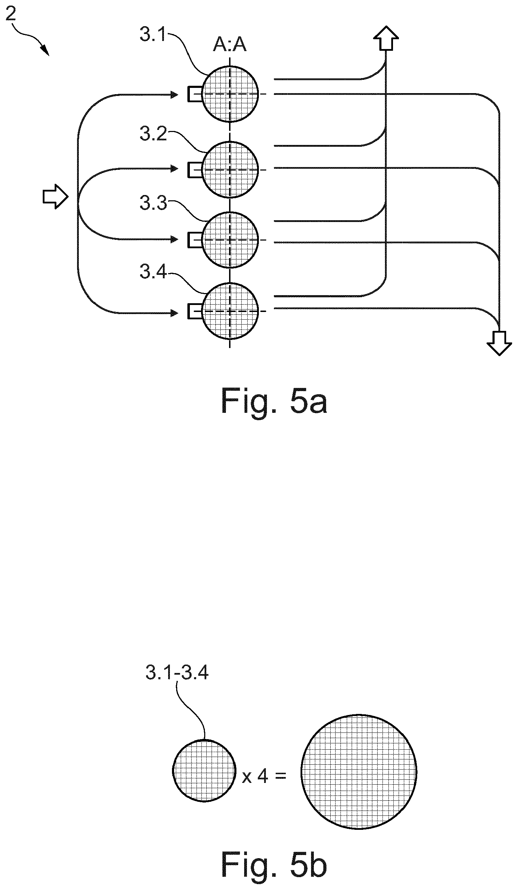

Fig. 5a zeigt eine Veranschaulichung einer zweiten Ausführungsform der Abscheideeinheit 2 der erfindungsgemäßen Anordnung 4 aus Fig. 1. Die Abscheideeinheit 2 weist vier strömungstechnisch parallelgeschaltete Abscheideeinrichtungen 3.1 bis 3.4 auf. Die Abscheideeinrichtungen 3.1 bis 3.4 sind identisch ausgebildet. Insbesondere ist also ein jeweiliger Strömungsquerschnitt der Abscheideeinrichtungen 3.1 bis 3.4 gleich groß und sind die Abscheideeinrichtungen 3.1 bis 3.4 für das gleiche Abscheideprinzip eingerichtet.

Die Aufteilung des Auslassstroms auf die Abscheideeinrichtungen 3.1 bis 3.4 in Abhängigkeit der Volumenströmungsrate des Auslassstroms kann in dieser Ausführungsform eingestellt werden, indem für die Abscheideeinrichtungen 3.1 bis 3.4 jeweils ein Anteil des Auslassstroms eingestellt wird, welcher durch die jeweilige Abscheideeinrichtung 3.1 bis 3.4 geleitet wird. Insbesondere kann eine Anzahl der Abscheideeinrichtungen 3.1 bis 3.4 eingestellt werden, durch welche jeweils zumindest ein Teil des Auslassstroms geleitet wird. Hat der Auslassstrom in einem ersten Fall eine kleinere Volumenströmungsrate als in einem zweiten Fall, kann der Auslassstrom im ersten Fall durch weniger der Abscheideeinrichtungen 3.1 bis 3.4 zumindest teilweise geleitet werden als im zweiten Fall.

Die Abscheideeinheit 2 aus Fig. 5a kann beispielsweise wie folgt betrieben werden:
▪ bei 15 bis 30 % einer Referenz-Volumenströmungsrate des Auslassstroms ist eine der Abscheideeinrichtungen 3.1 bis 3.4 aktiv,
▪ bei 30 bis 45 % einer Referenz-Volumenströmungsrate des Auslassstroms sind zwei der Abscheideeinrichtungen 3.1 bis 3.4 aktiv,
▪ bei 45 bis 60 % einer Referenz-Volumenströmungsrate des Auslassstroms sind zwei oder drei der Abscheideeinrichtungen 3.1 bis 3.4 aktiv - dies kann anhand eines weiteren Entscheidungskriteriums entschieden werden,
▪ bei 60 bis 90 % einer Referenz-Volumenströmungsrate des Auslassstroms sind drei oder vier der Abscheideeinrichtungen 3.1 bis 3.4 aktiv - dies kann anhand eines weiteren Entscheidungskriteriums entschieden werden,
▪ bei 90 bis 120 % einer Referenz-Volumenströmungsrate des Auslassstroms sind alle vier der Abscheideeinrichtungen 3.1 bis 3.4 aktiv.

Die Referenz-Volumenströmungsrate kann anhand einer Nennleistung festgelegt sein, welche auf bis zu 120 % davon überschritten werden kann.

Fig. 5b illustriert, dass der Strömungsquerschnitt einer einzelnen der Abscheideeinrichtungen 3.1 bis 3.4 mit einem Faktor vier multipliziert einem großen Gesamtströmungsquerschnitt entspricht. Sind alle vier Abscheideeinrichtungen 3.1 bis 3.4 aktiv, hat die Abscheideeinheit 2 die gleiche Funktion wie ein einfacher Abscheider mit einem Strömungsquerschnitt der in Fig. 5b rechts gezeigten Größe.

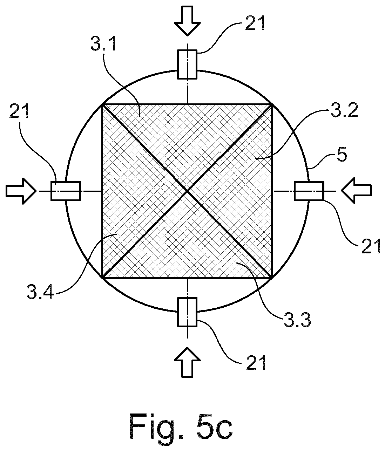

Fig. 5c zeigt mit einer Schnittdarstellung von oben, wie die Ausführungsform der Fig. 5a und 5b beispielsweise praktisch umgesetzt werden kann. Die vier Abscheideeinrichtungen 3.1 bis 3.4 sind in einem gemeinsamen Gehäuse 5 angeordnet. Jede der Abscheideeinrichtungen 3.1 bis 3.4 hat einen jeweiligen Einlass 21, so dass die Abscheideeinrichtungen 3.1 bis 3.4 einzeln angeströmt werden können.

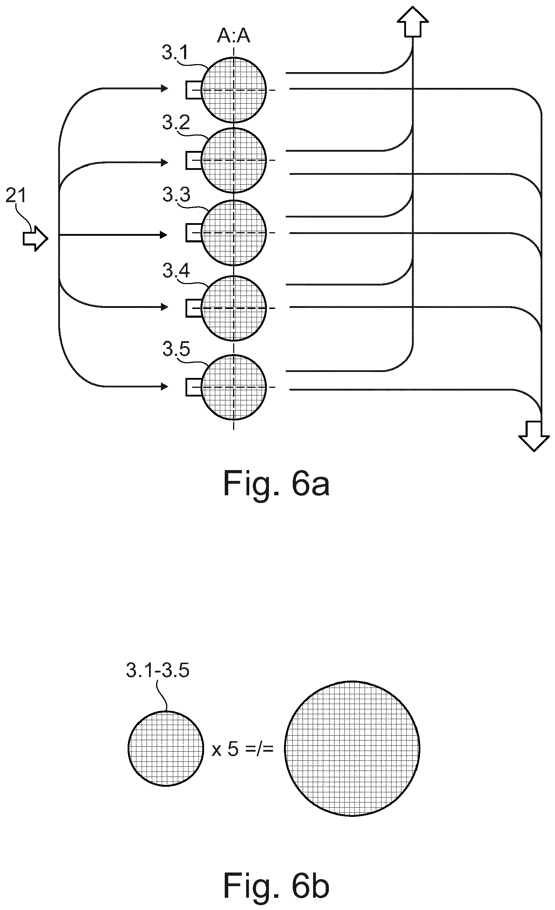

Fig. 6a und 6b zeigen analog zu den Fig. 5a und 5b eine dritte Ausführungsform der Abscheideeinheit 2 der erfindungsgemäßen Anordnung 4 aus Fig. 1. Im Falle der Fig. 6a und 6b ist jedoch zusätzlich eine fünfte Abscheideeinrichtung 3.5 vorgesehen.

Die Abscheideeinheit 2 aus Fig. 6a und 6b kann beispielsweise wie folgt betrieben werden:
▪ bei 15 bis 30 % einer Referenz-Volumenströmungsrate des Auslassstroms ist eine der Abscheideeinrichtungen 3.1 bis 3.4 aktiv,
▪ bei 30 bis 45 % einer Referenz-Volumenströmungsrate des Auslassstroms sind zwei der Abscheideeinrichtungen 3.1 bis 3.4 aktiv,
▪ bei 45 bis 60 % einer Referenz-Volumenströmungsrate des Auslassstroms sind zwei oder drei der Abscheideeinrichtungen 3.1 bis 3.4 aktiv - dies kann anhand eines weiteren Entscheidungskriteriums entschieden werden,
▪ bei 60 bis 75 % einer Referenz-Volumenströmungsrate des Auslassstroms sind drei oder vier der Abscheideeinrichtungen 3.1 bis 3.4 aktiv - dies kann anhand eines weiteren Entscheidungskriteriums entschieden werden,
▪ bei 75 bis 120 % einer Referenz-Volumenströmungsrate des Auslassstroms sind vier oder fünf der Abscheideeinrichtungen 3.1 bis 3.4 aktiv - dies kann anhand eines weiteren Entscheidungskriteriums entschieden werden,
▪ bei 120 bis 150 % einer Referenz-Volumenströmungsrate des Auslassstroms sind alle fünf der Abscheideeinrichtungen 3.1 bis 3.4 aktiv.

In diesem Fall wird davon ausgegangen, dass die Referenz-Volumenströmungsrate auf bis zu 150 % davon überschritten werden kann.

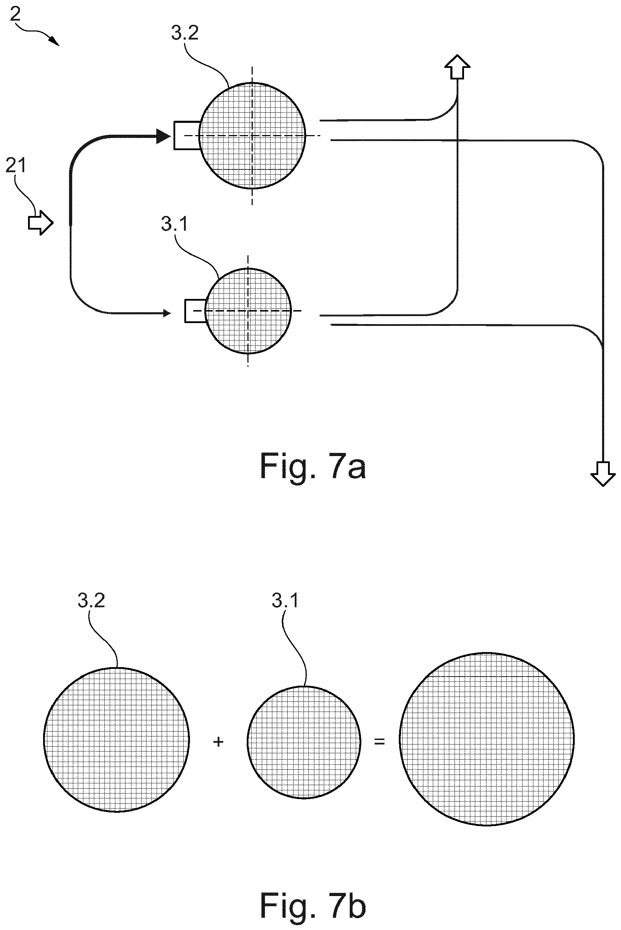

Fig. 7a und 7b veranschaulichen eine vierte Ausführungsform der Abscheideeinheit 2 der erfindungsgemäßen Anordnung 4 aus Fig. 1. In dem Fall hat die erste Abscheideeinrichtung 3.1 einen kleineren Strömungsquerschnitt als die zweite Abscheideeinrichtung 3.2. Angenommen, der Auslassstrom hat in einem ersten Fall eine kleineren Volumenströmungsrate als in einem zweiten Fall und in einem dritten Fall eine grö-βere Volumenströmungsrate als in dem zweiten Fall. Dann kann der Auslassstrom im ersten Fall vollständig durch die erste Abscheideeinrichtung 3.1 geleitet werden, im zweiten Fall vollständig durch die zweite Abscheideeinrichtung 3.2 geleitet werden und im dritten Fall jeweils teilweise durch die erste Abscheideeinrichtung 3.1 und durch die zweite Abscheideeinrichtung 3.2. Der dritte Fall ist in Fig. 7b veranschaulicht.

Die Abscheideeinheit 2 aus Fig. 7a und 7b kann beispielsweise wie folgt betrieben werden:
▪ bei 20 bis 40 % einer Referenz-Volumenströmungsrate des Auslassstroms ist nur die erste Abscheideeinrichtung 3.1 aktiv,
▪ bei 40 bis 60 % einer Referenz-Volumenströmungsrate des Auslassstroms ist nur die zweite Abscheideeinrichtung 3.2 aktiv,
▪ bei 60 bis 80 % einer Referenz-Volumenströmungsrate des Auslassstroms ist nur die zweite Abscheideeinrichtung 3.2 aktiv oder sind beide Abscheideeinrichtungen 3.1,3.2 aktiv - dies kann anhand eines weiteren Entscheidungskriteriums entschieden werden,
▪ bei 80 bis 120 % einer Referenz-Volumenströmungsrate des Auslassstroms sind beide Abscheideeinrichtungen 3.1,3.2 aktiv.

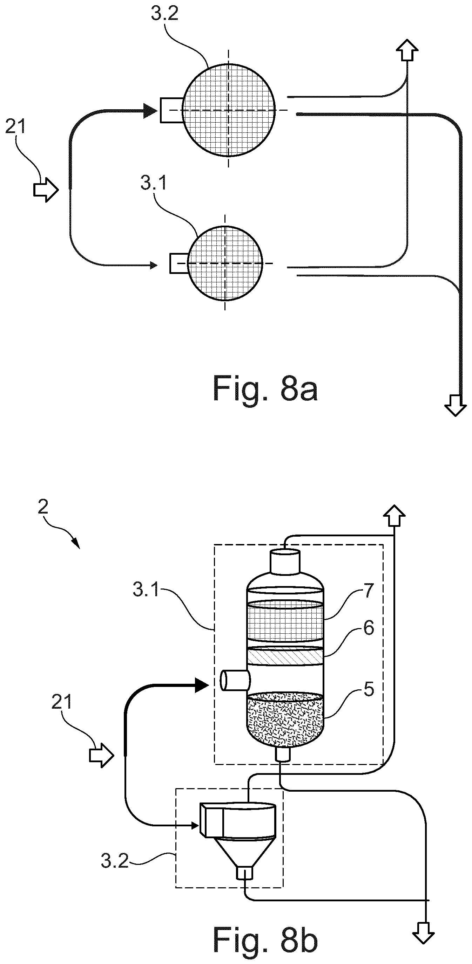

Fig. 8a und 8b veranschaulichen eine fünfte Ausführungsform der Abscheideeinheit 2 der erfindungsgemäßen Anordnung 4 aus Fig. 1. In dem Fall ist die erste Abscheideeinrichtung 3.1 für ein anderes Abscheideprinzip eingerichtet als die zweite Abscheideeinrichtung 3.2. Der Auslassstrom kann in dieser Ausführungsform analog zu der vierten Ausführungsform gemäß den Fig. 7a und 7b aufgeteilt werden.

Fig. 8b zeigt eine Möglichkeit, die fünfte Ausführungsform zu realisieren. Dabei ist die erste Abscheideeinrichtung 3.1 wie der aus dem Stand der Technik bekannte Abscheider aus Fig. 2a und 2b ausgebildet. Dazu ist allerdings die zweite Abscheideeinrichtung 3.2 parallelgeschaltet. In dem Fall ist die erste Abscheideeinrichtung 3.1 für ein anderes Abscheideprinzip eingerichtet als die zweite Abscheideeinrichtung 3.2. Die erste Abscheideeinrichtung 3.1 und die zweite Abscheideeinrichtung 3.2 sind zwar in unterschiedlichen Gehäusen ausgebildet, bilden aber dennoch gemeinsam die Abscheideeinheit 2.

### Bezugszeichenliste

- 1: Synthesereaktoranordnung
- 2: Abscheideeinheit
- 3.1: erste Abscheideeinrichtung
- 3.2: zweite Abscheideeinrichtung
- 3.3: dritte Abscheideeinrichtung
- 3.4: vierte Abscheideeinrichtung
- 3.5: fünfte Abscheideeinrichtung
- 4: Anordnung
- 5: Gehäuse
- 6: Vorabscheider
- 7: Hauptabscheider
- 8: Methanolauslass
- 9: Wasserzuleitung
- 10: CO₂-Quelle
- 11: H₂-Quelle
- 12: Rohgasquelle
- 13: erster Verdichter
- 14: zweiter Verdichter
- 15: Überdruckablass
- 16: erster Kühler
- 17: zweiter Kühler
- 18: Wärmetauscher
- 19: Dampftrommel
- 20: Dampfauslass
- 21: Einlass
- 22: Gasauslass
- 23: Flüssigkeitsauslass
- 24: Trennwand

## Patentansprüche

1. Verfahren zur Synthese von Methanol mit einer Synthesereaktoranordnung (1), wobei mit der Synthesereaktoranordnung (1) erzeugtes Methanol mit einer Abscheideeinheit (2) aus einem Auslassstrom der Synthesereaktoranordnung (1) abgeschieden wird, wobei die Abscheideeinheit (2) mehrere strömungstechnisch parallelgeschaltete Abscheideeinrichtungen (3.1 bis 3.5) aufweist, wobei der Auslassstrom derart auf die Abscheideeinrichtungen (3.1 bis 3.5) aufgeteilt wird, dass durch eine der Abscheideeinrichtungen (3.1 bis 3.5) der gesamte Auslassstrom geleitet wird oder durch mehrere der Abscheideeinrichtungen (3.1 bis 3.5) jeweils ein Teil des Auslassstroms geleitet wird, und wobei eine Aufteilung des Auslassstroms auf die Abscheideeinrichtungen (3.1 bis 3.5) in Abhängigkeit von einer Volumenströmungsrate des Auslassstroms eingestellt wird.

2. Verfahren nach Anspruch 1, wobei die Aufteilung des Auslassstroms auf die Abscheideeinrichtungen (3.1 bis 3.5) in Abhängigkeit der Volumenströmungsrate des Auslassstroms eingestellt wird, indem eine Anzahl der Abscheideeinrichtungen (3.1 bis 3.5) eingestellt wird, durch welche jeweils zumindest ein Teil des Auslassstroms geleitet wird.

3. Verfahren nach einem der vorhergehenden Ansprüche, wobei die Aufteilung des Auslassstroms auf die Abscheideeinrichtungen (3.1 bis 3.5) in Abhängigkeit der Volumenströmungsrate des Auslassstroms eingestellt wird, indem für die Abscheideeinrichtungen (3.1 bis 3.5) jeweils ein Anteil des Auslassstroms eingestellt wird, welcher durch die jeweilige Abscheideeinrichtung (3.1 bis 3.5) geleitet wird.

4. Verfahren nach einem der vorhergehenden Ansprüche, wobei der Auslassstrom in einem ersten Fall eine kleinere Volumenströmungsrate hat als in einem zweiten Fall, und wobei der Auslassstrom im ersten Fall durch weniger der Abscheideeinrichtungen (3.1 bis 3.5) zumindest teilweise geleitet wird als im zweiten Fall.

5. Verfahren nach einem der vorhergehenden Ansprüche, wobei der Auslassstrom in einem ersten Fall eine kleinere Volumenströmungsrate hat als in einem zweiten Fall, wobei eine erste der Abscheideeinrichtungen (3.1) einen kleineren Strömungsquerschnitt hat als eine zweite der Abscheideeinrichtungen (3.2), und wobei der Auslassstrom im ersten Fall vollständig durch die erste Abscheideeinrichtung (3.1) und im zweiten Fall vollständig durch die zweite Abscheideeinrichtung (3.2) geleitet wird.

6. Verfahren nach Anspruch 5, wobei der Auslassstrom in einem dritten Fall eine grö-βere Volumenströmungsrate hat als in dem zweiten Fall, und wobei der Auslassstrom im dritten Fall jeweils teilweise durch die erste Abscheideeinrichtung (3.1) und durch die zweite Abscheideeinrichtung (3.2) geleitet wird.

7. Anordnung (4) zur Synthese von Methanol umfassend eine Synthesereaktoranordnung (1) und eine an die Synthesereaktoranordnung (1) angebundene Abscheideeinheit (2), wobei die Abscheideeinheit (2) dazu eingerichtet ist, mit der Synthesereaktoranordnung (1) erzeugtes Methanol aus einem Auslassstrom der Synthesereaktoranordnung (1) abzuscheiden, und wobei die Abscheideeinheit (2) mehrere strömungstechnisch parallelgeschaltete Abscheideeinrichtungen aufweist (3.1 bis 3.5).

8. Anordnung (4) nach Anspruch 7, weiterhin aufweisend eine Steuereinheit, welche dazu eingerichtet ist, den Auslassstrom derart auf die Abscheideeinrichtungen (3.1 bis 3.5) aufzuteilen, dass durch eine der Abscheideeinrichtungen (3.1 bis 3.5) der gesamte Auslassstrom geleitet wird oder durch mehrere der Abscheideeinrichtungen (3.1 bis 3.5) jeweils ein Teil des Auslassstroms geleitet wird, und eine Aufteilung des Auslassstroms auf die Abscheideeinrichtungen (3.1 bis 3.5) in Abhängigkeit von einer Volumenströmungsrate des Auslassstroms einzustellen.

9. Anordnung (4) nach Anspruch 7 oder 8, wobei ein jeweiliger Strömungsquerschnitt der Abscheideeinrichtungen (3.1 bis 3.5) gleich groß ist.

10. Anordnung (4) nach Anspruch 7 oder 8, wobei eine erste der Abscheideeinrichtungen (3.1) einen kleineren Strömungsquerschnitt hat als eine zweite der Abscheideeinrichtungen (3.2).

11. Anordnung (4) nach einem der Ansprüche 7 bis 10, wobei die Abscheideeinrichtungen (3.1 bis 3.5) für das gleiche Abscheideprinzip eingerichtet sind.

12. Anordnung (4) nach einem der Ansprüche 7 bis 10, wobei eine erste der Abscheideeinrichtungen (3.1) für ein anderes Abscheideprinzip eingerichtet ist als eine zweite der Abscheideeinrichtungen (3.2).

13. Anordnung (4) nach einem der Ansprüche 7 bis 12, wobei zumindest einige der Abscheideeinrichtungen (3.1 bis 3.5) in einem gemeinsamen Gehäuse (5) angeordnet sind.

14. Anordnung (4) nach einem der Ansprüche 7 bis 13, wobei zumindest einige der Abscheideeinrichtungen (3.1 bis 3.5) in unterschiedlichen Gehäusen angeordnet sind.
